Europäisches Patentamt

European Patent Office

Office européen des brevets

(11) Publication number: **0 138 426 B1**

# EUROPEAN PATENT SPECIFICATION

(45) Date of publication of patent specification: 03.04.91

(51) Int. Cl.⁵: **C12N 15/31**, C12N 1/21, A01N 63/00, //(C12N1/21, C12R1:18,1:38,1:64)

(21) Application number: 84306462.7

(22) Date of filing: 21.09.84

(54) Ice nucleation deficient microorganisms by genetic manipulation.

(30) Priority: 22.09.83 US 534851

(43) Date of publication of application:
24.04.85 Bulletin 85/17

(45) Publication of the grant of the patent:
03.04.91 Bulletin 91/14

(84) Designated Contracting States:
AT BE CH DE FR GB IT LI LU NL SE

(56) References cited:
EP-A- 0 074 718
EP-A- 0 074 808

CHEMICAL ABSTRACTS, vol. 98, no. 7, 14th February 1983, page 1983, abstract no. 47935x, Columbus, Ohio, US; B. NIAUDET et al.: "Insertional mutagenesis in Bacillus subtilis: mechanism and use in gene cloning"

PROC. NATL. ACAD. SCI. USA, vol. 80, August 1983, pages 4894-4898; N.I. GUTTERSON et al.: "Replacement and amplification of bacterial genes with sequences altered in vitro"

(73) Proprietor: THE REGENTS OF THE UNIVERSITY OF CALIFORNIA
2199 Addison Street
Berkeley, California 94720(US)

(72) Inventor: Orser, Cindy S.
1540 Hearst Avenue
Berkeley California 94703(US)
Inventor: Lindow, Steven
2018 Virginia, No. C.
Berkeley California 94709(US)
Inventor: Panapoulos, Nickolas J.
4792 Belfast Street
Oakland California 94619(US)

(74) Representative: Harrison, David Christopher et al
MEWBURN ELLIS & CO 2/3 Cursitor Street
London EC4A 1BQ(GB)

"Molecular Genetics of the Bacteria-Plant Interaction", 1983, pages 353-361, edited by A. Pühler, Springer-Verlag, Berlin, DE; C. ORSER et al.: "Cloning of genes involved in bacterial ice nucleation and fluorescent pigment/siderophore production"

PHYTOPATHOLOGY, vol. 74, no. 7, 1984, page 798, ref. no. A66; C.S. ORSER et al.: "Structural and functional analysis of the pseudomonas syringae PV. Syringae ice region and construction of ice-deletion mutants"

NATURE, vol. 305, 22nd September 1983, page 262, Macmillan Journals Ltd.; P. DAVID: "How bacteria can protect plants"

FEDERAL REGISTER, 22.09.1982, Vol. 47, No. 184, FR 412925

## Description

Frost sensitive agricultural crops may be damaged by ice formation within their tissues. While water within the tissues may be supercooled to -5° C and below, the presence of certain natural epiphytic bacteria on the plant surface can promote the nucleation and formation of ice crystals at temperatures slightly below 0° C. Such ice nucleation capable (INA⁺) bacteria are responsible for frost damage in a wide variety of important agricultural crops, such as corn, soybeans, wheat, tomatoes, deciduous fruit trees such as pear, almond, apple, cherry, and many subtropical plants such as citrus and avocado.

Many approaches have been developed for reducing such frost injury. Methods such as the use of wind machines to mix warm air from above with the cold air near the ground, the burning of fossil fuels to directly heat the air surrounding the crop plants, and the pumping of large amounts of water to release latent heat on or near crop plants are not highly effective, are costly, and can adversely affect the environment. Alternate methods for frost control are actively being sought.

One effective approach relies on the reduction of the natural epiphytic population of ice nucleation capable bacteria on the crop plants. Approaches include the use of bactericides, typically antibiotics, to reduce the total bacterial population; and the use of antagonistic, ice nucleation deficient (INA⁻) microorganisms which compete with members of the natural epiphytic flora. The antagonistic INA⁻ strains heretofore have been selected from the natural populations of microorganisms which populate the plants, or have been generated by random mutagenesis of the naturally occurring INA⁺ strains. While use of such INA⁻ mutants has enjoyed success, such randomly-induced mutations are not always stable and are often accompanied by other mutations which debilitate the strain and reduce its ability to compete with the INA⁺ strains.

It would thus be desirable to develop I NA⁻strains from naturally-occurring INA⁺ bacteria, where the INA⁻ strains are stable and free from other randomly-introduced mutations.

U.S. Patent Nos. 4,045,910 and 4,161,084 describe the use of ice nucleation deficient microorganisms to inhibit frost injury. See also, copending application Serial No. 294,604 (EP-A-74718), filed August 20, 1981. A number of papers have been published concerning the effect of bacteria on ice nucleation. See, for example, Lindow et al., Proc. Am. Phytopathol. Soc. (1977) 4.1976:169; Arny et al., Nature (1976) 262:282-283; Lindow et al. Phytophathology (1978) 68:523-528; Lindow et al., Appl. Environ. Microbiol. (1978) 36:831-838; Lindow et al., Proc. Am. Phytopathol Soc. (1977) 3.1976:224. Orser et al in "Molecular Genetics of the Bacteria-Plant Interaction" (ed. A. Pühler), Springer-Verlag, 1983, describe the cloning of genes involved in bacterial ice nucleation. They describe how the cloned gene can confer ice nucleation activity on normally INA⁻ E.coli and on INA⁻ mutants of P.syringae and E.herbicola.

Notice of an application for permission to construct and release Pseudomonas syringae pv. syringae and Erwinia herbicola carrying in vitro generated deletions of all or part of the genes involved in ice nucleation was published in the Federal Register [47 FR 41925] September 22, 1982.

Methods and compositions are provided for inhibiting ice nucleation on plant hosts, particularly on agricultural crops. Ice nucleation deficient (INA⁻) microorganisms are provided by inducing non-reverting mutations within a genomic DNA sequence (INA gene) which encodes polypeptide(s) responsible for ice nucleation activity (INA). The modification of the genomic DNA is limited to the INA gene. Such microorganisms are genetically stable and free from deleterious mutations associated with random mutagenesis. The INA⁻ microorganisms of the present invention are applied to a plant part, preferably at an early stage of growth, so that they become established prior to the colonization of the plant part(s) by the INA⁺ microorganisms.

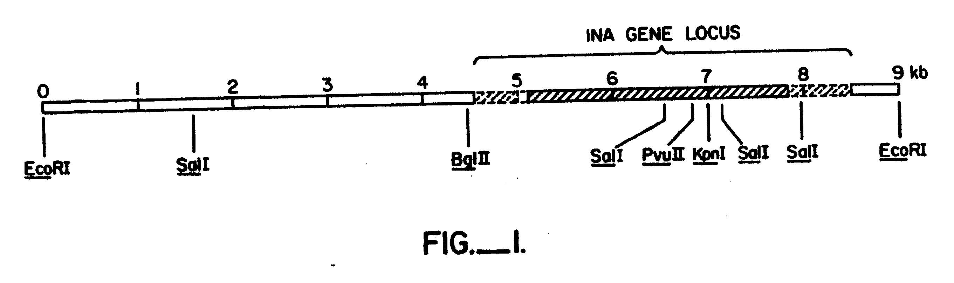

FIG. 1 illustrates the 9.0 kb Eco RI fragment of the genomic DNA of Pseudomenas syringae pv. syringae which carries the gene (ina ) responsible for INA.

Frost injury to host plants is inhibited by the application of ice nucleation deficient (INA⁻) microorganisms which proliferate on the surface of the plant and reduce the population of natural epiphytic ice nucleation capable (INA⁺) microorganisms. The INA⁻microorganisms are produced by in vitro modification of INA⁺ microorganisms which are naturally-present on the plant host of interest. Suitable modifications include deletions, substitutions, insertions, and the like, within the DNA locus (ina ) responsible for ice nucleation activity (INA), which modifications result in a non-reverting loss of INA. The INA⁻ microorganisms are applied to the host plant part at an early stage in the growth cycle, at or before the time when frost damage may occur.

INA⁺ microorganisms suitable for modification are those which populate the plant host of interest and which display the INA phenotype, i.e. ice nucleation at a temperature in the range of about -1.5° to -5.0° C. A specific test for identifying the INA⁺ phenotype is described below. Of particular interest are INA⁺ bacteria, including Pseudomonas species or pathovars such as syringae , cornofaciens , pisi , tabaci , garcae , glycinea and the like; Xanthamonas, such as translucens ; and Erwinia , such as herbicola .

Once a suitable strain of INA⁺ microorganisms has been selected, the strain will be converted to an INA⁻ phenotype by introducing a non-reverting alteration in the INA genetic region which confers the INA phenotype. The alteration should be substantially limited to the INA genetic region which may include from one to a plurality of structural genes as well as the regulatory regions which control expression of the polypeptide(s) responsible for the INA phenotype. In order to avoid mutation of other regions of the genomic DNA, in vitro gene manipulation methods are employed.

Suitable in vitro genetic manipulations include the introduction of deletions, substitutions, insertions, transversions, inversions, and the like within the INA gene. Usually, the manipulation will result in a deletion or substitution which affects at least about 5 base pairs of the DNA within the INA gene region.

In constructing the modified microorganisms of the present invention, it will be necessary to first identify and isolate the gene locus responsible for the INA phenotype. Conveniently, a genomic library may be obtained by restriction of the genomic DNA of the microorganism to provide fragments of suitable length. Various vectors, including plasmids, phages, and cosmids, can then be employed for cloning of the fragments. The vectors should provide a means for selection and/or screening, typically through antibiotic selection, packaging requirements, inactivation of a gene, and the like. Alternatively, a cDNA gene library may be prepared by first isolating the mRNA fraction from the source microorganisms. After extracting the total RNA, the mRNA is separated. The cDNA gene library can then be prepared using reverse transcriptase in the well known manner. The cDNA fragments are then cloned and screened in the manner just described.

Gene fragments carrying all or a portion of the INA gene region may be identified by their ability to confer ice nucleation activity on INA⁻ hosts. Conveniently, recombinant plasmids carrying the gene fragments can be screened by growing colonies of transformed hosts, typically E . coli or other INA⁻hosts, on a selective media. Viable colonies can be transferred to velvet pads which are replica-printed on sheets of aluminum foil precoated with a thin coating of paraffin. The foil is then adjusted from about -5° to -9°C, and the cells sprayed with atomized water droplets. Water which contacts INA⁺ cells freezes, giving a frosty appearance to the INA⁺ colonies. INA⁺ colonies can thus be identified.

Once the genomic fragments carrying the INA gene have been identified, the gene can be modified in a variety of ways and reintroduced into the parental strain to convert the parental strain to an INA⁻phenotype. Typically, the recombinant plasmid or other vector carrying the fragment will be mapped to locate the region comprising the INA gene and to identify restriction sites therein. The fragment may then be restricted or restricted and resected to remove at least a portion of the gene locus, including sequences of the structural gene(s) and/or regulatory control regions responsible for expression of the genes. The modified plasmid can then be introduced to the INA⁺ parental strain, and the deletion transferred by homologous recombination. It is desirable to use vectors incapable of replication in the microorganisms host so that the vector will be lost after recombination. Alternative, a vector which is a member of an incompatibility group, such as pRK290, may be used for marker exchange and thereafter eliminated by introduction of an incompatible vector. Such deletion mutations in the parental strain are substantially incapable of reversion to the INA⁺ phenotype.

In some cases, it may be desirable to insert a marker, such as resistance to biocides or cytotoxic agents, within the INA gene region or elsewhere within the microorganism genome. Insertion of the marker in the INA gene can be relied on to inactivate the INA gene, or can be done in combination with deletion and/or other mutations. The introduction of biocide or cytotoxic resistance can impart a competitive advantage to the resistant INA⁻ strains relative to the wild type INA⁺ strains. By applying the particular biocide or cytotoxic agent to the plant host at any stage of growth, particularly at an early stage, the resistant INA⁻ strains will predominate over the susceptible INA⁺. Such markers also allow monitoring of the proliferation of the modified microorganisms in the environment.

Alternatively, the INA⁺ gene region may be modified by transposon insertion, particularly one having a sequence homologous to a sequence present in the INA gene. A suitable transposon is inserted into the recombinant vector carrying the cloned INA gene locus. Vectors where the transposon is inserted within the INA gene locus are selected by loss of the INA⁺ phenotype. At least a portion of the inverted repeat at either end of the transposon is then excised, or other means employed, to prevent excision of the inserted sequence. The use of transposon insertions provides a convenient method for introducing antibiotic resistance to the modified microorganisms of the present invention.

In particular, a deletion mutation of Pseudomonas syringae pathovar syringae may be prepared as follows. The INA gene is found on an approximately 9.0 kb Eco RI fragment of the genomic DNA. This fragment is illustrated in FIG.

1. The INA gene comprises a sequence of about 3±1 kb. The gene includes three SalI and one KpnI restriction sites.

After digestion of the genomic DNA, the Eco RI fragment may be cloned and selected in any suitable vector. Conveniently, the fragment may be inserted at the unique Eco RI restriction site on pBR325. After transformation into E. coli HB101, recombinant plasmids may be selected based on tetracycline and/or ampicillin resistance, and INA$^+$ transformants scored by the ice nucleation test described above. The recombinant plasmids may then be modified, typically by partial digestion with Sal I. SalI partial digestion will produce deletions of about 1.0 or 1.5 kb within the INA gene locus, while leaving the flanking regions derived from the parental strain substantially intact. The digestion will also produce an approximately 6.1 kb deletion which includes a large portion of the flanking DNA. This latter deletion may be undesirable since it may affect other functions of the parental strain.

After identifying the plasmids carrying the 1.0 or 1.5 kb deletions by restriction mapping and gel electrophoresis, the deletion may be introduced into the parental strain by homologous recombination. Gutterson et al, PNAS, 80 , 4894-4898 (1983) disclose a method of replacement of bacterial genes with in vitro altered DNA by homologous recombination. The procedures described are of general applicability. The recombinant plasmid is introduced into the parental strain and a double cross-over event leads to mutants where substitution of the deleted INA gene locus on the plasmid for the wild-type locus on the genomic DNA of the parental strain has occurred. After the double cross-over, the recombinant pBR325 plasmid carrying the wild type INA gene region will be lost since it is unable to replicate in the pseudomonad host.

The modified microorganisms of the present invention may be utilized effectively in diverse formulations, including agronomically-acceptable adjuvants and carriers normally employed for facilitating the dispersion of active ingredients for agricultural applications. The precise formulations, dosage, mode of application and other variables are chosen to enhance the inhibition of ice nucleation activity in any given application. Thus, the previously described modified microorganisms may be formulated as a suspension or dispersion, aqueous or non-aqueous medium, as a dust, as a wettable powder, as an emulsifiable concentrate, as a granule, or as any of several known types of formulations, depending on the desired mode of application.

The concentration of cells in the formulation should be sufficient to provide for establishment of the cells on the host plant in competition with the naturally-present microorganisms. For wet formulations, e.g. foliage sprays, suspensions, aerosols, mists and the like, the concentration will generally be from about $10^5$ to $10^{10}$ cells per milliliter. For dry formulations, the concentration will range from about $10^4$ to $10^9$ cells per gram of formulated product.

The formulations may include various additives. In aqueous formulations, surfactants, nutrients, buffers, penetrating agents biological or chemical pesticides, and the like may be provided. In dry formulations, inert powders, bacterial stabilizing agents, salts, anti-caking agents, nutrients, buffers, film forming materials, biological or chemical pesticides, and the like may be provided. The additives will generally range in concentration from about $10^{-4}$ to 1 weight percent.

Preferably, the formulation of the present invention will be applied early in the growth stage of the plant host to provide for establishment of the ice nucleation deficient strains prior to substantial colonization by ice nucleation capable microorganisms. In the case of perennials, such as citrus crops, the formulation will be applied early in each growing season. For example, seeds, tuber parts, plantlets, propagants, or the like may be treated in conventional ways with a dispersion of the microorganisms of this invention in an appropriate medium, e.g., water, nutrient medium, etc. Alternatively, the host plant can be sprayed with a biocide in order to kill the ice nucleation capable microorganisms which are present prior to treatment. This will be particularly effective when the modified microorganisms have been provided with biocide resistance, as described above. In either case, the INA$^-$ strains of the present invention should be provided with a niche in which they may become established in order to inhibit the establishment of the INA$^+$ strains.

Although the foregoing invention has been described in some detail by way of illustration and example for purposes of clarity of understanding, it will be obvious that certain changes and modifications may be practiced.

## Claims

1. A process which comprises isolating from a normally INA$^+$ microorganism DNA containing an ina gene locus, carrying out in vitro modification of that DNA, substantially confined to a single ina gene locus, such as to impart loss of INA function to the gene, introducing the altered DNA into a normally INA$^+$ microorganism so as to replace a wild-type gene therein with the altered gene, and producing therefrom a culture of the microorganism which has lost said wild-type ina gene and thereby has acquired stable, non-reverting diminished ice-nucleation activity.

2. A process according to claim 1 wherein the altered DNA replaces the wild-type ina DNA by homologous recombination.

3. A process according to claim 2 wherein the altered ina gene is introduced into the INA⁺ microorganism on a vector which is incapable of replication in that microorganism, so that the vector is lost on culturing the microorganism.

4. A process according to claim 2 wherein the altered ina gene is introduced into the INA⁺ microorganism on a vector which is a member of an incompatibility group, and following the homologous recombination the vector containing the wild-type gene is eliminated by the introduction of an incompatible vector.

5. A process according to any one of claims 1 to 4 wherein said modification of the DNA comprises the deletion of at least a portion of the ina gene.

6. A mutant microorganism having stable, non-reverting diminished ice-nucleation activity, as obtainable by a process according to any one of claims 1 to 5.

7. A microorganism according to claim 6 selected from Pseudomonas syringae , Pseudomonas cornofaciens , Pseudomonas pisi , Pseudomonas tabaci , Pseudomonas fluorescens , Pseudomonas garcae , Pseudomonas glycinea , Xanthomonas translucens and Erwinia herbicola .

8. A method for inhibiting frost injury to a plant host susceptible to frost injury owing to the presence of ice-nucleation capable bacteria, said method comprising applying to at least a part of the plant host a mutant microorganism according to claim 6 or claim 7.

9. A method according to claim 8 wherein the mutant microorganism is applied to the plant host early in the growth stage or early in the growing season of the plant, to establish the mutant strain on the plant prior to substantial colonisation by wild-type INA⁺ microorganisms.

**Revendications**

1. Procédé qui comprend l'isolation à partir d'un microorganisme normalement INA⁺ j, d'un ADN contenant un locus du gène ina , la réalisation d'une modification in vitro de cet ADN, principalement réduit à un unique locus du gène ina , de telle manière à faire perdre la fonction INA au gène, en introduisant l'ADN altéré dans un microorganisme normalement INA⁺ de telle manière à remplacer le gène de type sauvage de celui-ci par un gène altéré, et la production à partir de celui-ci d'une culture du microorganisme qui a perdu le gène ina de type sauvage et a ainsi acquis une activité de formation de cristaux de glace diminuée non réversible et stable.

2. Procédé selon la revendication 1, où l'ADN altéré remplace l'ADN ina de type sauvage par une recombinaison homologue.

3. Procédé selon la revendication 2, où le gène ina altéré est introduit dans le microorganisme INA⁺ sur un vecteur qui est incapable de répliquer dans ce microorganisme, de telle manière à ce que le vecteur soit perdu dans la culture du microorganisme.

4. Procédé selon la revendication 2, où le gène altéré ina est introduit dans le microorganisme INA⁺ sur un vecteur qui est un membre d'un groupe d'incompatibilité, et suivant la recombinaison homologue, le vecteur contenant le gène de type sauvage est éliminé par l'introduction d'un vecteur incompatible.

5. Procédé selon l'une quelconque des revendications 1 à 4, où la modification précitée de l'ADN comprend la délétion d'au moins une partie du gène ina .

6. Microorganisme mutant ayant une activité stable de formation de cristaux de glace diminuée non réversible, tel qu'obtenu par le procédé selon l'une quelconque des revendications 1 à 5.

7. Microorganisme selon la revendication 6, sélectionné parmi Pseudomonas syringae , Pseudomonas cornofaciens , Pseudomonas pisi, Pseudomonas tabaci , Pseudomonas fluorescens , Pseudomonas garcae , Pseudomonas glycinea , Xanthomonas translucens et Erwinia herbicola .

8. Procédé pour éliminer les lésions du gel chez une plante hôte sensible aux lésions du gel dues à la présence de bactéries capables de former des cristaux de glace, le procédé précité comprenant l'application à au moins une partie de la plante hôte, d'un microorganisme mutant selon la revendication 6 ou la revendication 7.

9. Procédé selon la revendication 8, où le microorganisme mutant est appliqué sur la plante hôte de manière précoce dans la phase de croissance ou de manière précoce dans la saison de croissance de la plante, pour établir la souche mutante sur la plante avant une colonisation importante par des microorganismes INA$^+$ de type sauvage.

**Ansprüche**

1. Verfahren umfassend die Isolierung von DNA, die einen ina -Gen-Locus enthält, aus einem normalerweise INA$^+$-Mikroorganismus, die Durchführung der Modifizierung dieser DNA in vitro, die im wesentlichen auf einen einzigen ina -Gen-Locus beschränkt ist, um einen INA-Funktiensverlust im Gen zu induzieren, Einbringen der veränderten DNA in einen Mikroorganismus, der normalerweise INA$^+$ ist, um ein darin enthaltenes Wild-Typ-Gen durch das veränderte Gen zu ersetzen, und Herstellung einer Kultur des Mikroorganismus, der das genannte Wild-Typ ina -Gen verloren, und dadurch eine stabile, irreversible verringerte Fähigkeit zur Eiskernbildung erworben hat.

2. Verfahren nach Anspruch 1, worin die veränderte DNA die Wild-Typ ina -DNA durch homologe Rekombination ersetzt.

3. Verfahren nach Anspruch 2, worin das veränderte, ina -Gen in den INA$^+$-Mikroorganismus auf einem Vektor eingebracht wird, der unfähig zur Replikation in diesem Mikroorganismus ist, sodaß der Vektor beim Kultivieren des Mikroorganismus verlorengeht.

4. Verfahren nach Anspruch 2, worin das veränderte, ina -Gen in den INA$^+$-Mikroorganismus auf einem Vektor eingebracht wird, der Mitglied einer Inkompatibilitätsgruppe ist, und folgend auf die homologe Rekombination der das Wild-Typ-Gen enthaltende Vektor durch Einbringen eines inkompatiblen Vektors eliminiert wird.

5. Verfahren nach einem der Ansprüche 1 bis 4, worin die genannte Modifizierung der DNA die Löschung wenigstens eines Abschnittes des ina -Gens umfaßt.

6. Mutanter Mikroorganismus, der eine stabile, verringerte, irreversible Eiskernbildungsaktivität besitzt, wie er nach einem Verfahren nach einem der Ansprüche 1 bis 5 erhältlich ist.

7. Mikroorganismus nach Anspruch 6, ausgewählt aus Pseudomonas syringae, Pseudomonas cornofaciens, Pseudomonas pisi, Pseudomonas tabaci, Pseudomonas fluorescens, Pseudomonas garcae, Peusdomonas glycinae, Xanthomonas translucens und Erwinia herbicola.

8. Verfahren zur Hemmung von Frostschäden an einem Pflanzenwirt, der aufgrund der Gegenwart von zur Eiskernbildung fähigen Bakterien für Frostschäden anfällig ist, das genannte Verfahren umfassend das Aufbringen eines mutanten Mikroorganismus nach Anspruch 6 oder 7 auf wenigstens einen Teil des Pflanzenwirtes.

9. Verfahren nach Anspruch 8, worin der mutante Mikroorganismus auf den Pflanzenwirt in einem frühen Wachstumsstadium oder frühzeitig während der Wachstumsjahreszeit der Pflanze aufgebracht wird, um den mutanten Stamm auf der Pflanze vor einer wesentlichen Kolonienbildung durch Wild-Typ INA$^+$ Mikroorganismen anzusiedeln.

EP 0 138 426 B1

INA GENE LOCUS

0   1   2   3   4   5   6   7   8   9 kb

EcoRI   SalI   BglⅡ   SalI   PvuⅡ   KpnI   SalI   SalI   EcoRI

FIG.__I.